(19) **Europäisches Patentamt**

**European Patent Office**

**Office européen des brevets**

(11) **EP 1 413 250 B1**

(12) **EUROPEAN PATENT SPECIFICATION**

(45) Date of publication and mention
of the grant of the patent:
**29.03.2006 Bulletin 2006/13**

(51) Int Cl.:
*A61B 5/22* (2006.01)     *A61B 5/053* (2006.01)
*A61B 5/0488* (2006.01)     *G01G 19/50* (2006.01)
*G01G 19/414* (2006.01)

(21) Application number: **03023919.8**

(22) Date of filing: **21.10.2003**

(54) **Device for measurement of muscular volume**

Vorrichtung zur Messung des Muskelvolumens

Dispositif de mesure du volume musculaire

(84) Designated Contracting States:
**DE FR GB**

(30) Priority: **22.10.2002 JP 2002306829**

(43) Date of publication of application:
**28.04.2004 Bulletin 2004/18**

(73) Proprietor: **TANITA CORPORATION**
**Tokyo (JP)**

(72) Inventors:
• **Kouou, Takahito**
**Itabashi-ku**
**Tokyo (JP)**

• **Sakai, Yoshio**
**Itabashi-ku**
**Tokyo (JP)**

(74) Representative: **Müller-Boré & Partner**
**Patentanwälte**
**Grafinger Strasse 2**
**81671 München (DE)**

(56) References cited:
EP-A- 0 787 506          EP-A- 1 249 205
US-A- 4 540 002          US-A- 5 012 820
US-A1- 2001 053 883

• PATENT ABSTRACTS OF JAPAN vol. 1999, no.
12, 29 October 1999 (1999-10-29) -& JP 11 188016
A (OMRON CORP), 13 July 1999 (1999-07-13)

**Description**

BACKGROUND OF THE INVENTION

(i) Field of the Invention

The present invention relates to a device for making measurements with respect to muscles, i.e., determining the type of muscle and muscular fatigue of a subject.

(ii) Description of the Related Art

As a conventional method for measuring and determining the type of muscle fiber or muscular fatigue, a method of directly measuring a muscle tissue sample, a substance in the body such as lactic acid, a muscle pH or oxygen saturation in the blood and determining the type of muscle fiber or the muscular fatigue from the measurement value is known.

Further, an electromyogram is also known that detects a potential difference by use of electrodes set on the skin so as to measure an electrical signal delivered to move a muscle. Alternatively, a mechanomyogram is also available that detects minute vibrations on the surface of a muscle by use of piezoelectric elements. It is considered a signal reflecting the mechanical action of a muscle.

Further, a device is also known that calculates an FFM (Fat Free Mass) by use of a bioelectric impedance method and estimates the amount of a muscle from the calculated value.

Further, a device is disclosed that not only measures bioelectric impedance and calculates body fat but also measures a back strength by causing a subject to pull up a chain. This device is capable of measuring a muscular strength in addition to a body weight and body fat, see Japanese Patent Publication Laid-Open No. 321343/2001,

In conventionally known methods of measuring muscles, muscular fatigue and the type of muscle fiber, measurement devices using electromyograms are most frequently used. However, since all of these devices measure electromyograms after giving electric stimulation, the body of a subject is strained. Thus, it cannot be said that these devices are suitable for human bodies.

Further, to find out the type of muscle, it has been practiced that the maximum muscular strength of a subject is measured and the type of the muscle is determined from the measurement value and the data of an electromyogram or mechanomyogram. However, since the measurement of the maximum muscular strength is affected by such factors as physical and mental conditions, it is difficult for the subject to exert maximum power for every measurement. Further, this method requires training over a few days so as to measure the maximum muscular strength. Thus, the type of the muscle cannot be determined easily by this method.

Further, the device described in Japanese Patent Application Laid-Open No. 321343/2001 simply estimates the muscular strength of a subject from his power to pull up the chain at that time. However, such a device has no way to find out how much power the subject pulls up the chain with. That is, although the device does not know whether the subject pulls up the chain with his maximum muscular strength or about a half of the maximum muscular strength, the device determines that it is his current muscular strength. This involves the subject's subjective factor, and it is therefore cannot be said that the muscular strength is measured accurately.

US 4,540,002 discloses an electrical measuring system for the non-invasive examination of biological tissue.

It is the object of the invention to provide an improved muscle measuring device.

This object is fulfilled by a muscle measuring device having the features disclosed in claim 1. Preferred embodiments are defined in the dependent subclaims.

According to the present invention there is easily measured a maximum muscular strength which has heretofore been difficult to measure and make a muscle-related evaluation more accurately, more specifically, determined the proportions of the types of muscles in a particular portion of a subject and the occurrence of muscular fatigue and make an overall evaluation on the muscles.

SUMMARY OF THE INVENTION

Further, a muscle measuring device of the present invention comprises:

an input unit,
a bioelectric impedance measuring unit,
a calculation unit,
a load setting unit,
a mechanomyogram measuring unit, and

a determining unit,

wherein

the input unit inputs individual physical data,

the bioelectric impedance measuring unit measures bioelectric impedance,

the calculation unit calculates a muscle volume between portions to be measured of a subject from the input individual physical data and the measured bioelectric impedance and calculates a maximum voluntary contraction based on the calculated muscle volume,

the load setting unit sets a load to be imposed on a muscle based on the calculated maximum voluntary contraction,

the mechanomyogram measuring unit measures a mechanomyogram of the subject, and

the determining unit measures a mechanomyogram of a muscle of the subject when the subject does exercise with respect to the load, analyzes amplitudes and an average frequency from the time-series data of the measuredmechanomyogram, and determines the proportions of the types of muscles in the measured portions of the subject from the inflection points of the amplitude data and the average frequency data.

Further, in the muscle measuring device of the present invention, the types of muscles determined by the determining unit are a slow-twitch fiber and a fast-twitch fiber.

Further, in the muscle measuring device of the present invention, the types of muscles determined by the determining unit are an SO fiber, an FOG fiber and an FG fiber based on differences in biochemical metabolism properties.

Further, in the muscle measuring device of the present invention, the load setting unit changes the load stepwise during measurement of the mechanomyogram based on the calculated maximum voluntary contraction and changes a muscular strength exerted by the subject forcibly.

Further, the muscle measuring device of the present invention further comprises:

a muscular strength detecting unit,

a control unit, and

a display unit,

wherein

the muscular strength detecting unit detects a muscular strength exerted by the subject during measurement of the mechanomyogram,

the control unit calculates a difference in muscular strength which is a difference between the load set by the load setting unit and the subject's muscular strength detected by the muscular strength detecting unit, and

the display unit displays the difference in muscular strength, thereby making the muscular strength exerted by the subject more accurate.

Further, in the muscle measuring device of the present invention, the determining unit determines the occurrence of muscular fatigue by comparing the data of the amplitudes and average frequency of a mechanomyogram when a given load is imposed on a muscle of the subject with the data of the amplitudes and average frequency of a mechanomyogram analyzed in the past.

BRIEF DESCRIPTION OF THE DRAWINGS

Fig. 1 is a table showing classifications of the types of muscle fibers.

Fig. 2 is an oblique perspective view of the appearance of a muscle measuring device which is an embodiment of the present invention.

Fig. 3 is an internal block diagram of the muscle measuring device which is an embodiment of the present invention.

Fig. 4 is a main flow of the muscle measuring device which is an embodiment of the present invention.

Fig. 5 is a routine for determination of the types of muscles of the muscle measuring device which is an embodiment of the present invention.

Fig. 6 is a routine for determination of muscle fatigue of the muscle measuring device which is an embodiment of the present invention.

Fig. 7 is a diagram showing displayed results of the muscle measuring device which is an embodiment of the present invention.

Fig. 8 is a diagram showing other displayed results of the muscle measuring device which is an embodiment of the present invention.

Fig. 9 is a diagram showing the constitution of a cuff of the muscle measuring device which is an embodiment of the present invention.

Fig. 10 is a schematic diagram showing a mechanomyogram when an exerted muscular strength is increased.
Fig. 11 is a diagram showing a subject pulling up a bar.
Fig. 12 is a schematic diagram showing the relationship between the RMS amplitude and average frequency of a mechanomyogram and a muscular strength.
Fig. 13 is another display example of the muscle measuring device which is an embodiment of the present invention.
Fig. 14 is another display example of the muscle measuring device which is an embodiment of the present invention.
Fig. 15 is another display example of the muscle measuring device which is an embodiment of the present invention.
Fig. 16 is another display example of the muscle measuring device which is an embodiment of the present invention.

Reference numeral 1 denotes a measuring device; 2 a scale equipped with a body fat meter; 2a a platform; 3 and 4 an electrode section; 3a, 4a, 13a and 14a an electric current supply electrode; 3b, 4b, 13b and 14b a voltage measuring electrode; 5 an operation box; 6 an input unit; 7 a display unit; 8 a print unit; 13 and 14 an electrode grip; 15, 16 and 24 a code; 17 a hook; 21 a bar; 22 a chain; 23 a cuff; 25 a muscle sound measuring unit; 30 an electrode switching unit; 31 an electric current supply unit; 32 a voltage measuring unit; 33 a control unit; 34 a storage unit; 35 a clocking unit; 36 a body weight measuring unit; 38 a power unit; 41 a muscular strength detection unit; and 42 a load control unit.

DETAILED DESCRIPTION OF THE PREFERRED EMBODIMENT

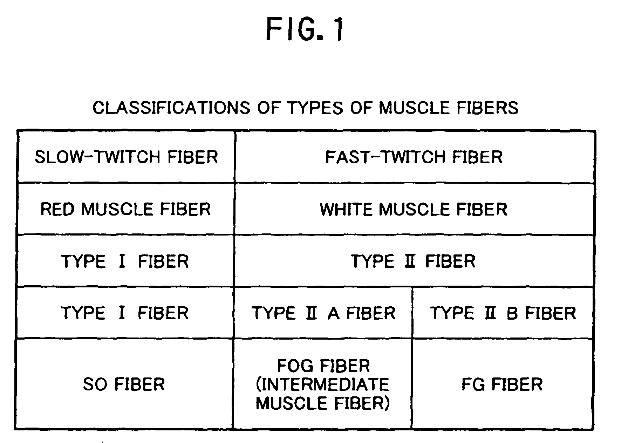

As shown in Fig. 1, the types of muscle fibers are classified by a variety of names due to differences among classification methods.

The most popular histochemical classification method at present is a Myosin ATPase staining method. A fiber stained dark is classified as a Type 2 fiber, and a fiber not stained dark is classified as a Type 1 fiber. The Type 1 fiber is also referred to as a slow-twitch fiber due to its low contraction speed to electric stimulation, while the Type 2 fiber is also referred to as a fast-twitch fiber due to its high contraction speed.

Further, muscle fibers are classified into three types of fibers, i.e., an FG (Fast-twitch Glycolytic) fiber having a high contraction speed and an excellent glycolytic ability, an FOG (Fast-twitch Oxidative) fiber having a high contraction speed and excellent glycolytic and oxidative abilities, and an SO (Slow-twitch Oxidative) fiber having a low contraction speed and an excellent oxidative ability, based on differences in biochemical metabolism properties.

Further, muscular tissues are roughly classified into three types, i.e., "smooth muscles" which are distributed in internal organs and blood vessel walls, "heart muscles" which constitute the muscle layers of the heart, and "skeletal muscles" which are solely allowed to move voluntarily under control of encephalon nerves.

The mechanism of the "skeletal muscle" (muscle fiber) which moves voluntarily is such that it generates an action potential upon receipt of stimulation from motor nerves, contracts, and causes tension. At this time, oxygen in the blood is consumed. However, when intense muscle contractions persist for a long time, a reduction in bloodstream and/or absence of oxygen conditions partially occur, so that lactic acid is produced. Accordingly, the contractile force lowers. This condition is referred to as muscular fatigue.

Whether one is liable to feel the muscular fatigue associates with his muscular endurance. The muscular endurance refers to an ability of moving muscles by an aerobic energy supply mechanism. One with muscular endurance can supply aerobic energy over a long time. Consequently, lactic acid is not liable to be accumulated, so that he is not liable to feel muscular fatigue. On the other hand, one without muscular endurance is liable to feel muscular fatigue.

Exertion of muscular strength is controlled by three factors, i.e., the number of muscle cells (number of MU) used in action of muscles, the frequency of excitation of motor nerves, and the types of muscle fibers.

An electromyogram is a waveform formed by overlapping of the action potentials of muscle fibers, and a mechanomyogram represents vibrations caused by contraction of muscle on receipt of the action potentials.

As a load imposed on a muscle is increased, the amount of nerve impulses given to the muscle is increased, and along with that, an increase in the number of MU and an increase in muscle fibers occur, whereby the frequencies and amplitudes of an electromyogram and mechanomyogram increase. As the muscular strength further increases, all MUs are used. Further, when the muscular strength reaches at least 80% of maximum muscular strength, a further muscular strength must be exerted, so that the frequency of the impulses increases. As a result, the frequency and amplitude of the electromyogram increase. However, while the frequency of the mechanomyogram increases, its amplitude decreases. This is assumed to be because the muscle vibrates (tetanic contraction) in a fully stretched state.

The present invention utilizes the foregoing characteristics of muscles, measures bioelectric impedance by use of a plurality of electrodes which are brought into contact with various parts of a living body, and calculates muscle volumes and maximum voluntary contractions in the parts such as hands and feet of the subject from the measured bioelectric impedance.

Further, based on the calculated maximum voluntary contraction, the amount of a load to be imposed on the subject is adjusted, a mechanomyogram when different amounts of loads are imposed on the muscles is measured by use of piezoelectric elements, the frequency data of the mechanomyogram is analyzed so as to obtain data on an average frequency and amplitudes, the amounts of actions (delivery frequencies) of muscle fibers are calculated from inflection points thereof, and the types of muscles (muscle fibers) and muscular fatigue of the subject are determined from these measurements.

Example

An embodiment of the present invention will be described with reference to the drawings.

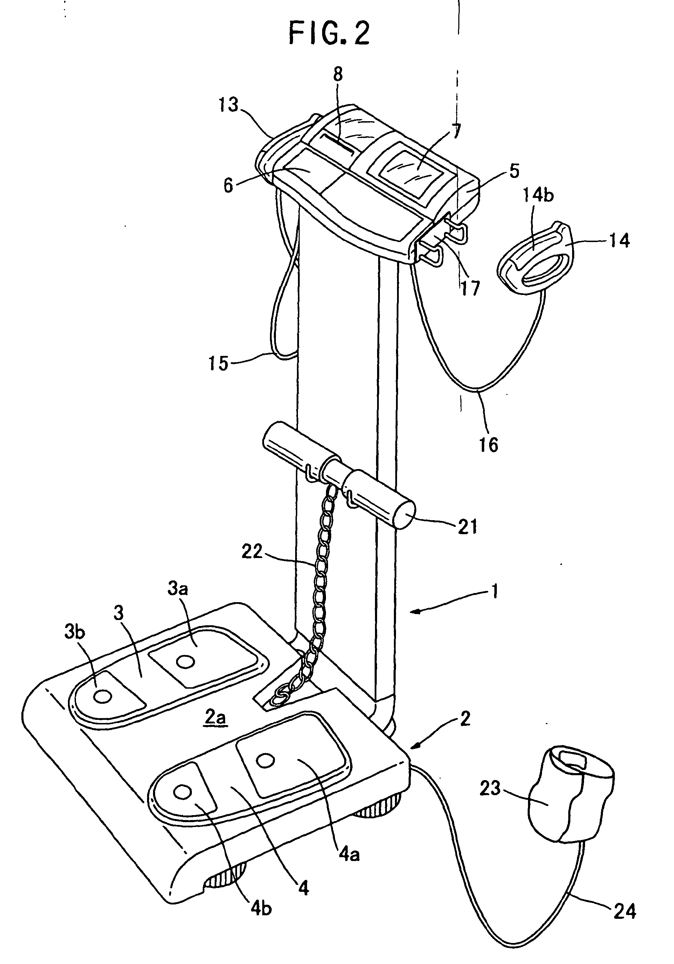

Fig. 2 is an oblique perspective view of the appearance of a muscle measuring device which is an embodiment of the present invention. The measuring device 1 is nearly L-shaped. At the bottom of the device 1 is provided a scale 2 equipped with a body fat meter. The scale 2 equipped with a body fat meter is a known device, and electrode sections 3 and 4 which make contact with the sole of both feet of a subject are provided on the surface of a platform 2a on which the subject stands to measure his body weight. The electrode sections 3 and 4 comprise electric current supply electrodes 3a and 4a and voltage measuring electrodes 3b and 4b.

Further, on the top surface of the measuring device 1, an operation box 5 is provided. The operation box 5 comprises a power switch, an input unit 6 as input means for inputting various physical data, a display unit 7 as display means comprising an LCD for display measurement results, and a print unit 8 which prints the measurement results on a sheet of paper and ejects the paper.

Further, to the operation box 5, electrode grips 13 and 14 for hands are connected via cords 15 and 16. The electrode grips 13 and 14 for hands also comprise electric current supply electrodes 13a and 14a and voltage measuring electrodes 13b and 14b. While not used for measurement, the electrode grips 13 and 14 for hands are hooked on hooks 17 which are provided on both sides of the operation box 5.

Further, to the front side of the scale 2 equipped with a body fat meter, a bar 21 as load means for imposing a load on a muscle is connected by a chain 22. The bar 21 is hooked on hooks.

In addition, a cuff 23 which is attached to a part such as a leg or arm where a measurement of muscle is made is connected to the scale 2 by means of a cord 24. In the cuff 23, a transducer (sensor) 25 which is a device for measuring muscle sounds and two electrodes 23a and 23b for measuring bioelectric impedance are provided.

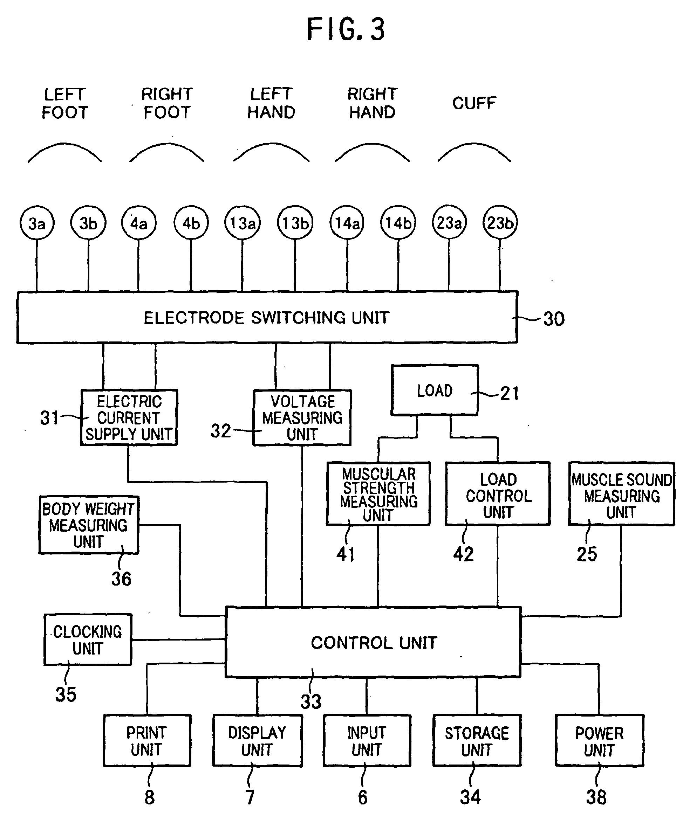

Fig. 3 is an electrical block diagram of the measuring device 1. The ten electrodes 3a, 3b, 4a, 4b, 13a, 13b, 14a, 14b, 23a and 23b which make contact with both hands and feet as measuring means are connected to an electrode switching unit 30. The electrode switching unit 30 is connected to a control unit 33 as control means via an electric current supply unit 31 and a voltage measuring unit 32. The control unit 33 has a microcomputer (CPU) to perform various computations and controls. To the control unit 33, a storage unit 34 which comprises a memory or register as storage means for storing various data, a clocking unit 35 for clocking given time and a body weight measuring unit 36 for measuring the body weight of a subject are connected. Further, the input unit 6, the display unit 7 and the print unit 8 are also connected to the control unit 33. A power unit 38 supplies power to the control unit 33 and other units.

Further, the measuring device 1 also has a muscular strength detection unit 41 for detecting a muscular strength to pull up the bar 21 and a load control unit 42 for controlling the load of the bar 21.

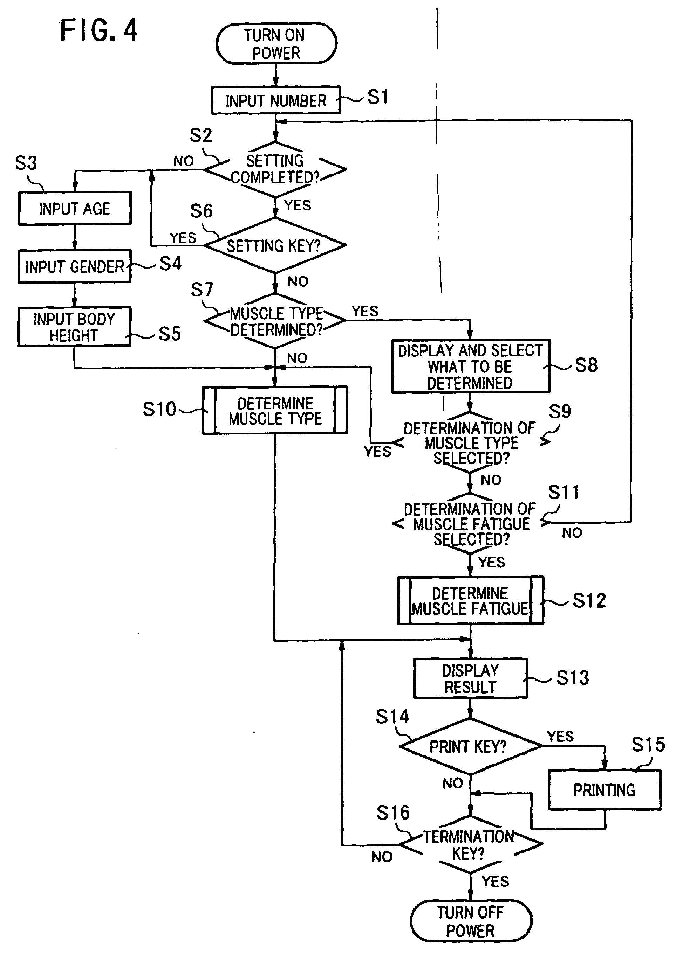

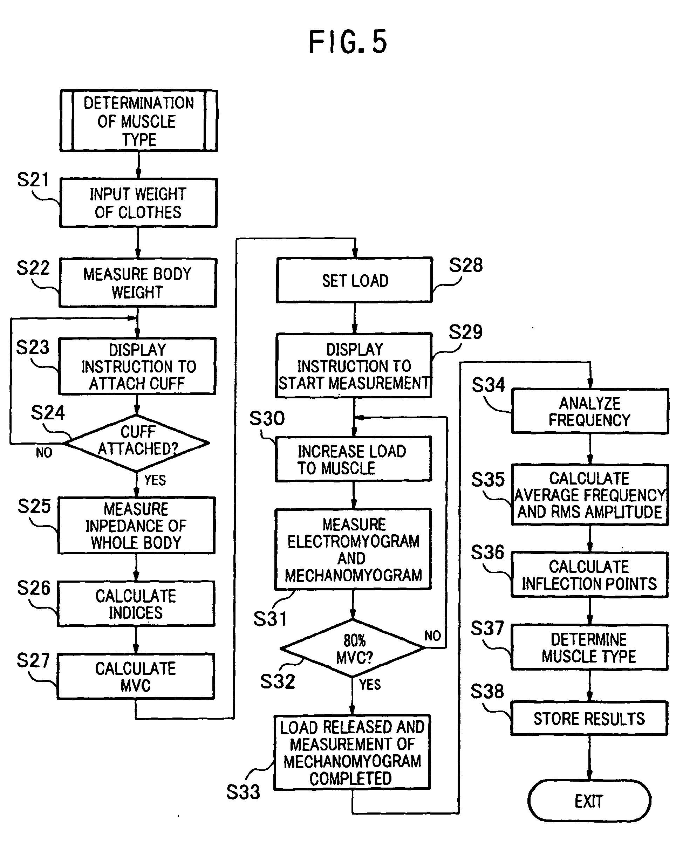

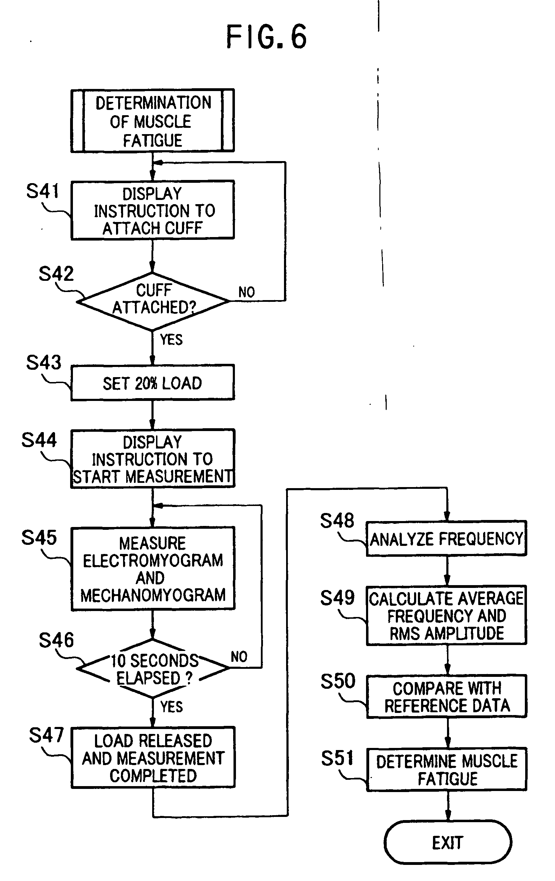

Next, the operation of the measuring device 1 of the present invention will be described by use of the flowcharts of Figs. 4 to 6. Further, switches and keys in the present invention refer to those provided in the input unit 6, and at the press of these switches and keys, data and numeric values can be entered.

Firstly, at the press of the power switch provided in the input unit 6 of the measuring device 1, all electrical units are initialized, and the measuring device 1 waits for a number to be entered therein. At this point, a subject enters his personal administration number (STEP S1).

It is checked if there is already personal data set in a memory area in the storage unit 34 which corresponds to the entered personal number. If not, the measuring device 1 is forced into a mode of setting personal data (STEP S2).

As the personal data, age, gender and a body height are entered (STEPS S3 to S5).

Further, even if the personal data is already set, it is confirmed whether the subject is attempting to change the set personal data by checking whether a setting key is pressed or not. The measuring device 1 also enters the setting mode when the setting key is pressed (STEP S6).

If the setting key is not pressed down in STEP S6, it is checked whether determination of the type of muscle is already completed (STEP S7).

If the determination of the type of muscle is already completed at this point, the subject can select making the determination of the type of muscle or determination of muscle fatigue and the display unit 7 displays the selectable items (STEP S8).

If the determination of the type of muscle is selected (STEP S9) or if the determination of the type of muscle is completed in STEP S7 after completion of the settings in STEPS S4 to S6, the measuring device 1 enters a muscle type determination mode (STEP S10). This muscle type determination mode will be described later.

Then, if the determination of muscle fatigue is selected (STEP S11), the measuring device 1 enters a muscle fatigue determination mode (STEP S12).

After the determination of the type of muscle and the determination of muscle fatigue are made, the results are shown on the display unit 7 (STEP S13).

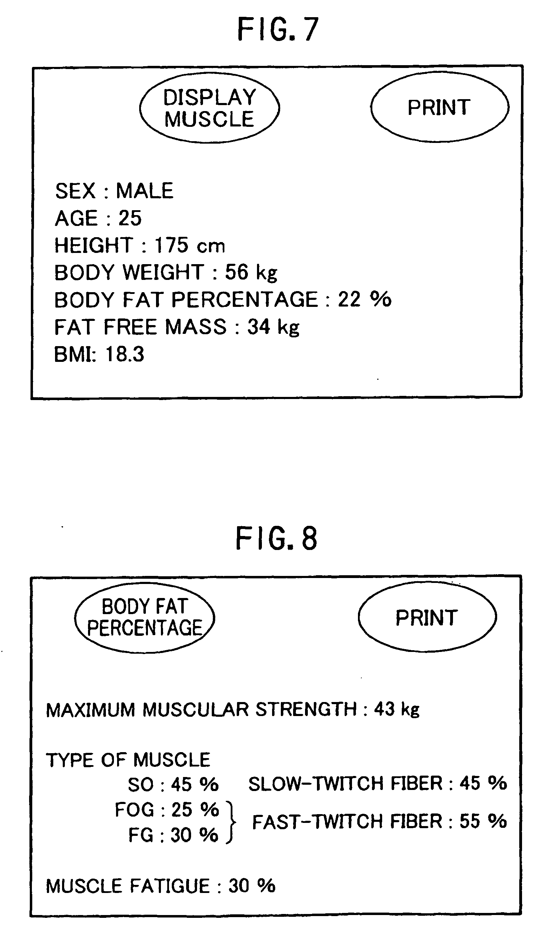

Fig. 7 and 8 show examples of displayed results. Fig. 7 shows the results of measuring general physical characteristics. Fig. 8 shows the results of measuring a maximum muscular strength, the type of muscle and the degree of muscle fatigue. The words displayed in the upper portions of these screens indicate inputtable keys. In Fig. 7, at the press of the muscle display key, the screen image is switched to that shown in Fig. 8, while the screen image is switched to that shown in Fig. 7 at the press of the fat percentage key.

If the print key is pressed (STEP S14), the print unit 8 prints the results on paper and ejects the paper (STEP S15).

If an end key is pressed, the power is turned off, whereby the device stops. Until the end key is pressed, the results displayed in STEP S13 are kept displayed.

<Muscle Type Determination Mode>

The muscle type determination mode is a mode for determining balance between fast-twitch fibers and slow-twitch fibers in a muscle in a part to be measured of a subject by measuring a mechanomyogram.

Firstly, the subject enters the weight of clothes by use of the input unit 6 (STEP S21).

Then, the body weight of the subject is measured. The subject stands on the scale 2 so as to measure the body weight by use of the body weight measuring unit 36 (STEP S22). The measured body weight value is stored in the storage unit 34.

Then, an instruction urging the subject to attach the cuff 23 to a part where a measurement of muscle is made is displayed on the display unit 7 (STEP S23).

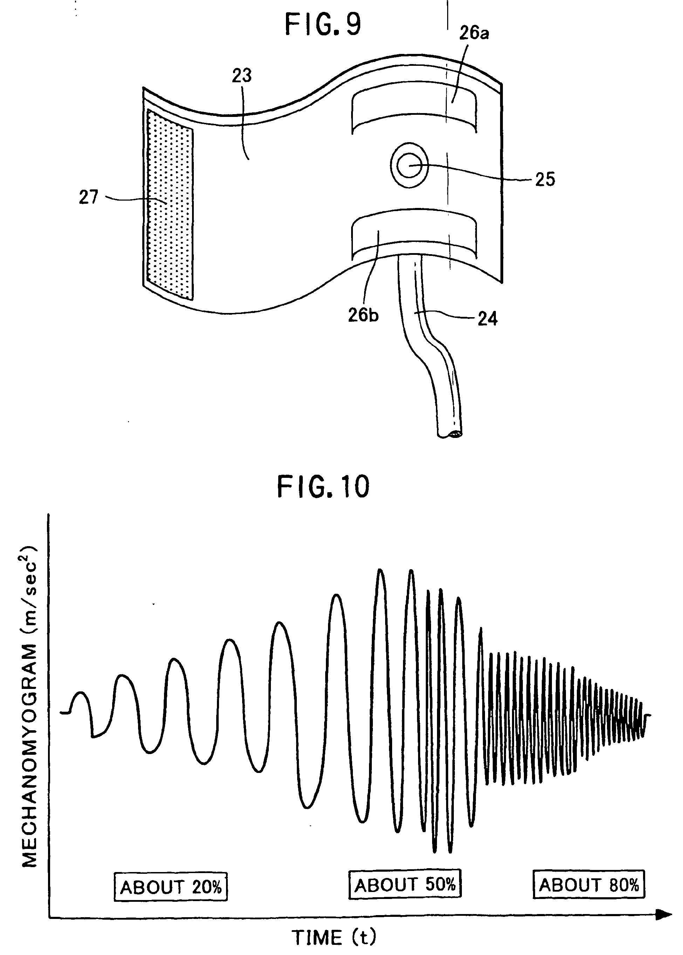

The cuff 23 has a constitution as shown in Fig. 9. On the internal surface of the main body of the cuff, electrodes 26a and 26b are provided. Further, between the electrodes, a vibration sensor (transducer) 25 for measuring muscle sounds is provided. At both ends of the cuff 23, magic tapes (registered trademark) 27 are provided. By use of these magic tapes, the cuff is tied and fixed around a part to be measured.

After finished attaching the cuff 23 to the part to be measured, the subject enters completion of the attachment (STEP S29). In this case, it is assumed that the cuff is attached to the thigh of the subject's right leg.

Then, the subject enters the circumference of the part to be measured. Since the cuff 23 has graduations on the external surface along the circumferential direction, the subject can determine the circumference with the cuff tied and fixed around the part to be measured, and the subject enters the circumference by use of numeric keys.

Then, the impedance of the whole body is measured (STEP S25).

Urged by an instruction displayed on the display unit 7. to grip the grips 13 and 14, the subject grips the grips 13 and 14. The subject should grip the grips 13 and 14 such that the electrodes 13a, 13b, 14a and 14b make contact with his palms.

The electrodes to be connected to the electric current supply unit 31 and the voltage measuring unit 32 are switched in turn by the electrode switching unit 30 so as to switch parts to be measured.

Further, the impedance of the part to be measured is also measured.

Because the muscle of the thigh of the right leg is measured in this case, an electric current passes between the electric current supply electrodes 4a and 14a, i.e., through the right leg, and a measurement of voltage is made by use of the electrodes 23a and 23b provided in the cuff 23.

A body mass index (BMI) is calculated by use of the body weight measured in STEP S22 and set personal data. Further, a body fat percentage is calculated by use of the measured impedance value, the body weight value and the set personal data (STEP S26) . Descriptions of these calculation methods will be omitted since they are known in the art.

Further, a part muscle volume is calculated by use of the impedance Zp of the measured part.

In this case, based on an expression for calculating a fat free mass FFM, a muscle volume MV is calculated based on the following expression by use of Ht as a body height, W as a body weight and Age as age.

$$MV = a_1 WZp/Ht^2 + b_1 Z + c_1 Age + d_1$$

wherein $a_1$, $b_1$, $c_1$ and $d_1$ are coefficients varying depending on gender.

When a fat free mass (fat free proportion) containing a large amount of muscle fibers is large, a muscular strength is exerted easily, so that a maximum voluntary contraction increases. Further, in general, females have a high body fat percentage, while males have a low body fat percentage. At the same age, body height and body weight, a lower fat percentage inevitably ensures a larger muscle volume present in the body. Accordingly, it is considered that the maximum voluntary contraction increases according to those parameters as well.

Therefore, by use of the calculated MV, a maximum voluntary contraction MVC is calculated based on the following expression:

$$MVC = a_2MV + b_2Age + c_2$$

wherein $a_2$, $b_2$ and $c_2$ are coefficients varying depending on gender.

By the above calculation, the maximum voluntary contraction MVC is calculated (STEP S27).

After calculation of the maximum voluntary contraction MVC, the amount of a load to be imposed on the subject is calculated based on the calculated value. In this case, the amount of a load with which a muscular strength of 80% (MVC of 80%) is exerted is calculated from the maximum voluntary contraction data of the subject. The amount of the load is controlled such that there is no load at the start of the measurement and the amount of the load is gradually increased to reach the calculated load amount after passage of a given time.

This is because a mechanomyogram becomes as shown in Fig. 10 as an exerted muscular strength is gradually increased. Fig. 10 schematically shows time on the horizontal axis and a mechanomyogram on the vertical axis. When the subject exerts a low muscular strength of about 20%, the amplitude and the frequency are both small. However, when the subject exerts a medium muscular strength of about 50%, both the amplitude and the frequency become large, and when the subject exerts a maximum muscular strength of higher than 80%, the amplitude becomes smaller, but the frequency further increases. By increasing the amount of the load automatically so as to obtain such a phenomenon as data of an electromyogram, the subject is forced to exert muscular strengths of about 20% to about 80%, during which a mechanomyogram is measured.

Therefore, a program for the amount of the load is set based on the MVC (STEP S28) . In this case, the program is set such that the amount of the load is gradually increased from zero to a load amount corresponding to an MVC of 80% over 15 seconds.

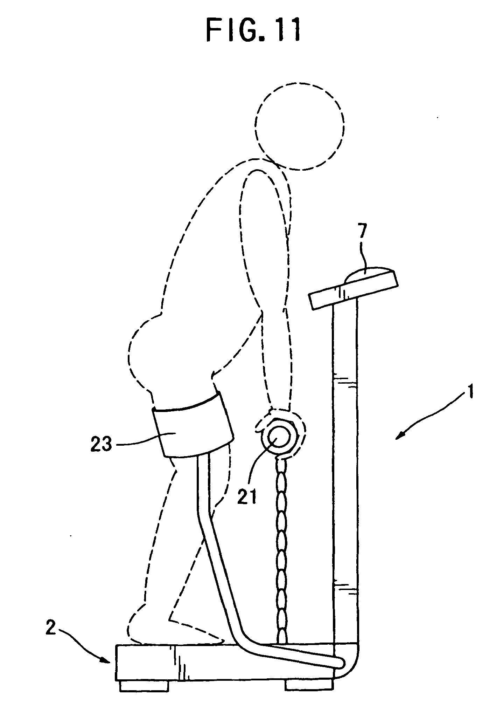

At this point, an instruction urging the subject to grip and pull up the bar 21 is displayed on the display unit 7 (STEP S29). Fig. 11 is a diagram showing the subject gripping and pulling up the bar 21. Firstly, the subject pulls the bar 21 upward with his knees bent at about 110°. At this point, particularly the thigh muscles of the thighs of both legs of the subject are tensed. Since the cuff 23 is tied around the thigh of the right leg, a mechanomyogram of the thigh muscle of the right leg is measured. At that time, a proper pull-up value is displayed on the display unit 7. This proper value indicates whether the subject is pulling up the amount of the load at that time accurately. The muscular strength measuring unit 41 as muscular strength detection means measures a strength pulling up the bar 21. When pulling is weak, a negative value is displayed, while when pulling is hard, a positive value is displayed. A proper value of "0" notifies the subject that he is pulling up the amount of the load properly.

The amount of a load imposed on the bar 21 is controlled by the load control unit 42 so as to gradually increase (STEP S30). Therefore, when the bar 21 is kept pulled up with a constant strength, the proper value becomes negative. Consequently, as the subject keeps pulling up the bar 21 so as to keep the proper pull-up value at "0", an exerted muscular strength naturally increases along with an increase in the amount of the load.

A mechanomyogram is measured by the muscle sound measuring unit (vibration sensor) 25 as muscle sound measuring means (STEP S31). At this point, it is checked whether 80% MVC has been exceeded (STEP S32), the amount of the load decreases, and the measurement of the mechanomyogram is completed (STEP S33).

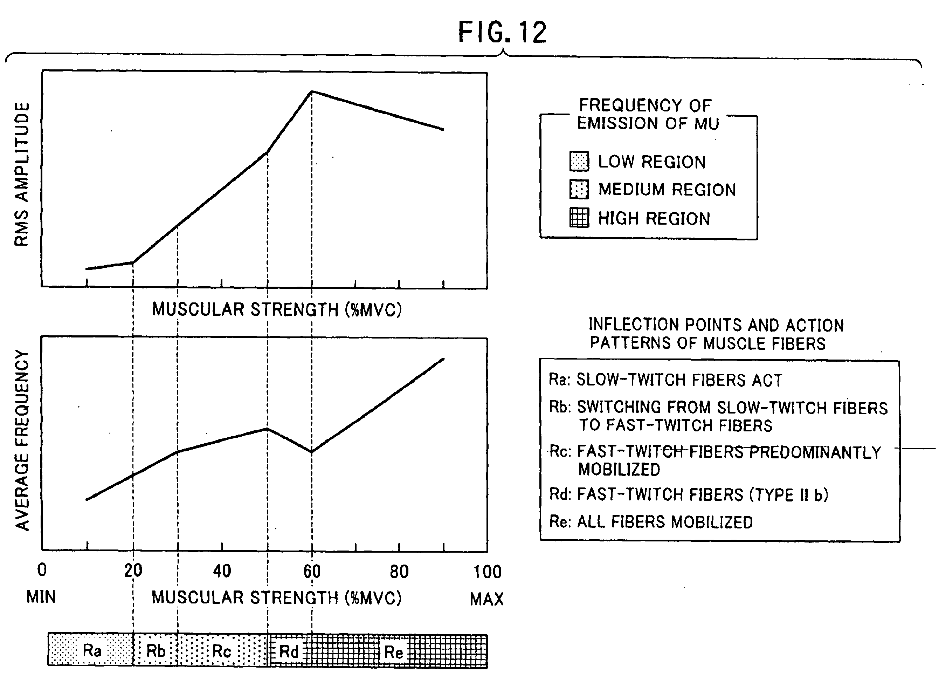

Fig. 12 is a schematic diagram showing the amplitude and average frequency of a muscle sound waveform after the muscle sound waveform is power spectrum analyzed at various percentages (%MVC) of the maximum voluntary contraction (MVC). An RMS (root mean square) amplitude basically represents the number of MU (including FG, SO and FOG) required to be mobilized into muscle action. While the value of the RMS amplitude becomes larger as the muscular strength increases, the value becomes smaller in the mechanomyogram when a tetanic contraction occurs. This is because when a high muscular strength is to be exerted, tension occurs in a muscle, so that the contraction and tension of the muscle do not occur easily.

Meanwhile, the average frequency represents the frequency of emission of impulses (in this case, signals to exert a muscular strength) . Referring to the RMS amplitude and the average frequency in Fig. 12, SO fibers are activated at 20% MVC or lower. At around 20 to 30% MVC, the SO fibers are switched to FOG fibers, whereby the RMS amplitude sharply increases and an inflection point occurs. At around 30 to 50% MVC, the FOG fibers are predominantly mobilized, inflection points occur in the average frequency. At about 50 to 60% MVC, FG fibers are also mobilized in need of a larger contraction, so that the average frequency which is emission frequency decreases, and the RMS amplitude which is the number of MU becomes slightly steeper. At 60% MVC or higher, all fibers are mobilized and a tetanic contraction occurs, so that the RMS amplitude decreases. Further, since the impulse further increases, the average frequency increases. By finding the inflection points, the proportions of the types of muscles can be calculated.

In the present invention, the proportions of the types of muscles are determined by use of these two indices.

Thus, the data of the calculated mechanomyogram is subjected to time-frequency analysis (Fourier transform or Wavelet transform) so as to calculate RMS amplitudes and average frequencies at various %MVC (STEP S35) and also calculate inflection points in the data of the RMS amplitude and average frequency (STEP S36). The inflection points can be determined by subjecting plot data to first derivation and extracting a minimum value.

By referring to the data of the determined inflection points of the RMS amplitude and average frequency and %MVC at that time, the proportions of actions of muscles. In the present invention, the proportions of actions of muscles are equal to the proportions of the types of muscles a subject has, and the proportions of the types of muscles the subject has are calculated (STEP S37). More specifically, as shown in the bar graph shown at the bottom of Fig. 12, the proportions of mobilized muscles can be determined. As shown by this bar graph, a muscular strength spectrum is divided into 5 regions. The characteristics of these regions (action patterns of muscle fibers) are as shown in Fig. 12. Ra represents action of slow-twitch fibers (Type 1 or SO fibers), Rb represents switching from the slow-twitch fibers to fast-twitch fibers (Type 2 A or FOG fibers), Rc represents predominant mobilization of the fast-twitch fibers, Rd represents fast-twitch fibers (Type 2 B or FG fibers), and Re represents mobilization of all fibers.

By use of the bar graph data shown at the bottom of Fig. 12, the proportions of the types of muscles are calculated in the following manner.

$$\text{Proportion of SO Fibers (\%)} = \text{\%MVC to Rb}$$

$$\text{Proportion of FOG Fibers (\%)} = \text{\%MVC to Rc} - \text{\%MVC to Ra}$$

$$\text{Proportion of FG Fibers (\%)} = \text{\%MVC to Rd} - \text{\%MVC to Rc}$$

The thus calculated proportions of the muscle fibers are %values based on mobilized muscles and do not make up 100% even if added together. Therefore, by calculating values divided by the total of the proportions of all muscle fibers, more specifically, by performing the following calculations, the proportions of the types of muscles in a measured part are calculated.

$$\text{Proportion of SO Fibers (\%)} = \text{Amount of SO Fibers/Total of Proportions of All Muscle Fibers X 100}$$

$$\text{Proportion of FOG Fibers (\%)} = \text{Amount of FOG Fibers/Total of Proportions of All Muscle Fibers X 100}$$

$$\text{Proportion of FG Fibers (\%)} = \text{Amount of FG Fibers/Total of Proportions of All Muscle Fibers X 100}$$

Further, as shown in Fig. 1, the slow-twitch fibers and the fast-twitch fibers are related to the SO fibers, the FOG fibers and the FG fibers. Thus, the proportions of the slow-twitch fibers and the fast-twitch fibers are also calculated as follows.

$$\text{Slow-Twitch Fibers} = \text{SO Fibers,}$$

Fast-Twitch Fibers = FOG Fibers + FG Fibers

In the present invention, the proportions of the types of muscles are determined by the above processes.

The determined proportions of the types of muscles of the subject are stored in the storage unit 34 (STEP S38).

Next, the muscle fatigue determination mode will be described.

The muscle fatigue determination refers to determination of how much fatigue has occurred at that point. The muscle fatigue is determined by which type of muscle is used when a certain load is imposed.

In the muscle fatigue determination mode, an instruction urging the subject to attach the cuff 23 to a part where muscle fatigue is determined is displayed on the display unit 7 (STEP S41).

After attaching the cuff 23 to a part to be measured, the subject enters completion of the attachment (STEP S92).

Then, setting of a load is carried out. The MVC of the subject stored in the storage unit 34 is retrieved, 20% MVC is calculated, and the load is set and controlled by the load control unit 42 so as to exert the 20% MVC (STEP S43).

Then, an instruction urging the subject to grip and pull up the bar 21 is displayed on the display unit 7 (STEP S44) .

At this time as well, the subject keeps pulling up the bar 21 so as to keep a proper pull-up value of "0", and a mechanomyogram at this time is measured (STEP 45).

This measurement continues for 10 seconds. It is checked whether time measured by the clocking unit 35 exceeds 10 seconds (STEP 46). Upon passage of 10 seconds, the load decreases, and the measurement of the mechanomyogram is ended (STEP S47). Then, the data of the measured mechanomyogram is power spectrum analyzed (STEP S48) so as to determine an average frequency and an average RMS amplitude value during the 10 seconds (STEP S49).

These average values are checked against the average frequency and RMS amplitude of the subject and data on the proportions of the types of muscles determined from the inflection points of the average frequency and RMS amplitude which are stored in the storage unit (STEP S50), and muscle fatigue is determined by how much % of MVC has been required to keep pulling up the load corresponding to the 20% MVC. That is, the occurrence of the muscle fatigue is determined by determining which types of muscles have been mobilized (STEP S51).

For example, a load to cause the subject to exert a muscular strength corresponding to 20% MVC is set, a mechanomyogram when the subject pulls up the load is measured, and the data of the mechanomyogram is frequency analyzed. When the analyzed data is compared with the past muscle fiber data (data shown in Fig. 12) of the subject which is stored in the storage unit and the amplitude and frequency are an amplitude and frequency corresponding to 50% MVC, muscle fatigue is determined to be 30% from 50% - 20% = 30%.

Alternatively, when the muscular strength corresponding to 20% MVC of the subject is normally classified in Rb and the measured average values of the average frequency and RMS amplitude fall within the range of Rc, this means that muscles corresponding to Rc have been mobilized to pull up this time's load, implying the occurrence of muscle fatigue. Meanwhile, when the calculated values fall within the normal ranges of the frequency and RMS amplitude in the muscular strength spectrum, this indicates that only normal muscles have been mobilized. Hence, it may be determined that there is no occurrence of muscle fatigue.

In addition to the foregoing embodiment of the present invention, a function of calculating and displaying a muscle cross sectional area of a part where a measurement of muscle is made may also be provided.

Further, a plurality of cuffs for making measurements on parts may be provided so as to measure muscles of multiple parts in a single load measurement.

Further, a load to be imposed on a muscle is not limited to the foregoing type of pulling up the bar and may also be, for example, a type of applying a grip to a palm such as a grip dynamometer, a type of pulling up a load by the legs or a type comprising a combination of these.

Further, the impedance measuring means which has been described as an integral device comprising a plurality of electrodes which can make contact with both hands and feet may be any device as long as it is capable of determining a maximum voluntary contraction of a part to be measured. Thus, the electrode to be attached to a part to be measured may be a clip-type electrode, and the present invention is still achievable even if an electric current supply electrode is provided in the cuff.

Further, an electromyogram may also be measured in addition to a measurement of mechanomyogram. The amplitude and frequency data resulting from frequency analyzing the data of an electromyogram when the muscular strength exerted by a subject is gradually increased also have inflection points. Thus, it is considered possible to correct inflection points determined in a mechanomyogram.

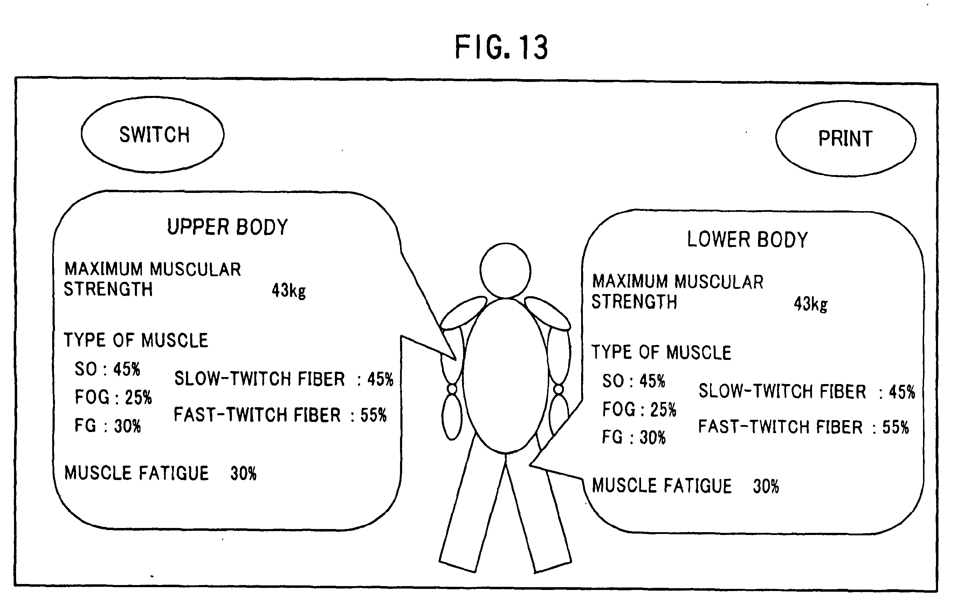

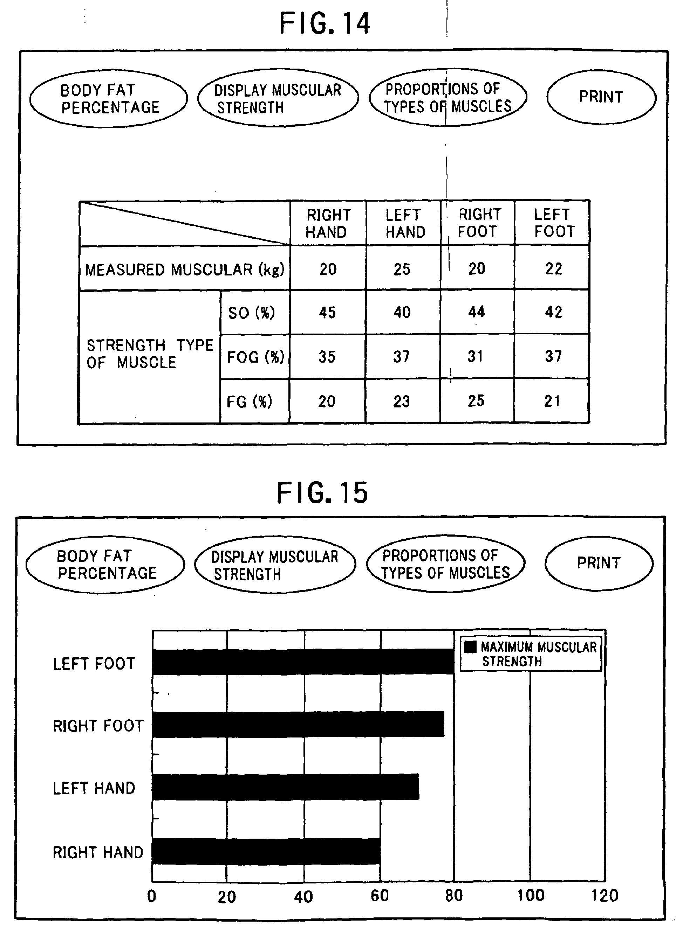

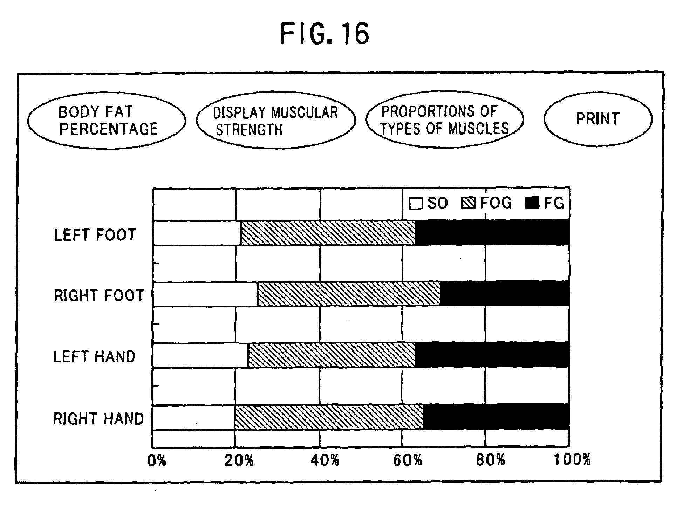

Further, as examples of displayed measurement results, those shown in Figs. 13 to 16 are also conceivable. Fig. 13 displays indices associated with the muscles of the upper and lower bodies, thereby allowing a subject to know a balance between the muscles of the upper and lower bodies. Fig. 14 displays the muscular strengths of both hands and feet and the proportions of muscle fibers constituting the muscles of the body parts as numeric values, thereby allowing a subject to specifically know the constituents of the muscles of the body parts. Fig. 15 shows the maximum muscular strengths of both hands and feet by means of graphs, thereby allowing a subject to visually understand the muscular strengths of the body parts. Fig. 16 displays the proportions of muscle fibers constituting the muscles of both hands and feet by means of graphs, thereby allowing a subject to visually understand the constituents of the muscles of the body parts.

According to the muscle measuring device of the present invention, it calculates a maximum voluntary contraction of a subject by measuring bioelectric impedance and determining the proportions of the types of muscle fibers constituting a measured part by analyzing the data of the mechanomyogram of the subject. Thus, a maximum voluntary contraction which has heretofore included a subject's subjective factor can be calculated objectively and easily, and a mechanomyogram can be measured accurately.

Further, according to the muscle measuring device of the present invention, a load is changed automatically so that the muscular strength exerted by the subject is forcibly changed based on the calculated maximum voluntary contraction. Therefore, the time-series data of a mechanomyogram when an exerted muscular strength is changed can be acquired easily.

Further, according to the muscle measuring device of the present invention, it detects a muscular strength which is actually being exerted by the subject during measurement of a mechanomyogram, compares the muscular strength exerted by the subject with a set load and displays the difference. Thus, the muscular strength exerted by the subject is always a proper value, and the time-series data of a mechanomyogram required to determine the proportions of muscle fibers become easier to obtain.

Further, according to the muscle measuring device of the present invention, it determines muscle fatigue by comparing mechanomyogram data at normal time which has been analyzed in the past and the current measured mechanomyogram data. Thus, muscle fatigue in a measured part can be known objectively and easily, thereby making the muscle measuring device of the present invention useful.

**Claims**

1. A muscle measuring device comprising:

    an input unit (6),
    a bioelectric impedance measuring unit (3, 4, 31, 41), and
    a calculation unit (33),
    wherein
    the input unit (6) inputs individual physical data,
    the bioelectric impedance measuring unit (3, 4, 31, 41) measures bioelectric impedance, and
    the calculation unit (33) calculates a muscle volume between portions to be measured of a subject from the input individual physical data and the measured bioelectric impedance and calculates a maximum voluntary contraction based on the calculated muscle volume,
    **characterized in that** the device further comprises
    a load setting unit (21, 42),
    a mechanomyogram measuring unit (25), and
    a determining unit (33),
    wherein
    the load setting unit (21, 42) sets a load to be imposed on a muscle based on the calculated maximum voluntary contraction,
    the mechanomyogram measuring unit (25) measures a mechanomyogram of the subject, and
    the determining unit (33) measures a mechanomyogram of a muscle of the subject when the subject does exercise with respect to the load, analyzes amplitudes and an average frequency from the time-series data of the measured mechanomyogram, and determines the proportions of the types of muscles in the measured portions of the subject from the inflection points of the amplitude data and the average frequency data.

2. The device of claim 1, wherein the types of muscles determined by the determining unit (33) are a slow-twitch fiber and a fast-twitch fiber.

3. The device of claim 1 or 2, wherein the types of muscles determined by the determining unit (33) are an SO fiber, an FOG fiber and an FG fiber based on differences in biochemical metabolism properties.

4. The device of one of claims 1 to 3, wherein the load setting unit (21, 42) changes the load stepwise during measurement of the mechanomyogram based on the calculated maximum voluntary contraction.

5. The device of one of claims 1 to 4, further comprising:

a muscular strength detecting unit (41),
a control unit (33), and
a display unit (7),
wherein
the muscular strength detecting unit (41) detects a muscular strength exerted by the subject during measurement of the mechanomyogram,
the control unit (33) calculates a difference in muscular strength which is a difference between the load set by the load setting unit (21, 42) and the subject's muscular strength detected by the muscular strength detecting unit (41), and
the display unit (7) displays the difference in muscular strength.

6. The device of one of claims 1 to 5, wherein the determining unit (33) determines the occurrence of muscular fatigue by comparing the data of the amplitudes and average frequency of a mechanomyogram when a given load is imposed on a muscle of the subject with the data of the amplitudes and average frequency of a mechanomyogram analyzed in the past.

**Patentansprüche**

1. Muskelmeßvorrichtung, umfassend:

eine Eingabeeinheit (6),
eine Meßeinheit (3, 4, 31, 41) der bioelektrischen Impedanz, und
eine Berechnungseinheit (33),
wobei
die Eingabeeinheit (6) individuelle physikalische Daten eingibt,
die Meßeinheit (3, 4, 31, 41) der bioelektrischen Impedanz eine bioelektrische Impedanz mißt, und
die Berechnungseinheit (33) ein Muskelvolumen zwischen zu messenden Bereichen eines Subjekts bzw. einer Person aus den eingegebenen individuellen physikalischen Daten und der gemessenen bioelektrischen Impedanz berechnet und eine maximale freiwillige bzw. spontane Kontraktion basierend auf dem berechneten Muskelvolumen berechnet,
**dadurch gekennzeichnet, daß** die Vorrichtung weiters umfaßt
eine Lastfestlegungseinheit (21, 42),
eine Mechanomyogram-Meßeinheit (25), und
eine Bestimmungseinheit (33),
wobei
die Lastfestlegungseinheit (21, 42) eine Last bzw. Belastung festlegt bzw. einstellt, die auf einen Muskel basierend auf der berechneten maximalen spontanen Kontraktion aufzubringen ist,
die Mechanomyogram-Meßeinheit (25) ein Mechanomyogram des Subjekts mißt, und
die Bestimmungseinheit (33) ein Mechanomyogram eines Muskels des Subjekts mißt, wenn das Subjekt in bezug auf die Last übt, Amplituden und eine mittlere Frequenz aus den Zeitseriendaten des gemessenen Mechanomyograms analysiert und die Proportionen der Arten von Muskeln in den gemessenen Abschnitten des Subjekts aus den Flektions- bzw. Wendepunkten der Amplitudendaten und den Daten der mittleren Frequenz bestimmt.

2. Vorrichtung nach Anspruch 1, wobei die Arten von Muskeln, die durch die Bestimmungseinheit (33) bestimmt sind, eine langsam zuckende Faser und eine schnell zuckende Faser sind.

3. Vorrichtung nach Anspruch 1 oder 2, wobei die Arten von Muskeln, die durch die Bestimmungseinheit (33) bestimmt sind, eine SO Faser, eine FOG Faser und FG Faser basierend auf Unterschieden in biochemischen Metabolismuseigenschaften sind.

4. Vorrichtung nach einem der Ansprüche 1 bis 3, wobei die Lastfestlegungseinheit (21, 42) die Last stufenweise während einer Messung des Mechanomyograms basierend auf der berechneten maximalen spontanen Kontraktion verändert.

**5.** Vorrichtung nach einem der Ansprüche 1 bis 4, weiters umfassend:

eine Muskelstärke-Detektionseinheit (41),
eine Steuer- bzw. Regeleinheit (33), und
eine Anzeigeeinheit (7),
wobei
die Muskelstärke-Detektionseinheit (41) eine Muskelstärke detektiert, die durch das Subjekt während der Messung des Mechanomyograms ausgeübt wurde,
die Steuer- bzw. Regeleinheit (33) einen Unterschied in der Muskelstärke berechnet, welcher ein Unterschied zwischen der Last, die durch die Lastfestlegungseinheit (21, 42) festgelegt ist, und der Muskelstärke des Subjekts ist, die durch die Muskelstärke-Detektionseinheit (41) detektiert ist, und
die Anzeigeeinheit (7), die die Differenz in der Muskelstärke anzeigt.

**6.** Vorrichtung nach einem der Ansprüche 1 bis 5, wobei die Bestimmungseinheit (33) das Auftreten von Muskelermüdung durch ein Vergleichen der Daten der Amplituden und mittleren Frequenz eines Mechanomyograms wenn eine gegebene Last auf einen Muskel des Subjekts aufgebracht ist, mit den Daten der Amplituden und der mittleren Frequenz eines Mechanomyograms bestimmt, das in der Vergangenheit analysiert wurde.

## Revendications

**1.** Dispositif de mesure de muscle comprenant :

une unité d'entrée (6),
une unité de mesure d'impédance bioélectrique (3, 4, 31, 41), et
une unité de calcul (33),
dans lequel
l'unité d'entrée (6) entre des données physiques individuelles,
l'unité de mesure d'impédance bioélectrique (3, 4, 31, 41) mesure l'impédance bioélectrique, et
l'unité de calcul (33) calcule un volume musculaire entre des parties à mesurer d'une personne à partir des données physiques individuelles entrées et de l'impédance bioélectrique mesurée et calcule une contraction volontaire maximale sur la base du volume musculaire calculé,
**caractérisé en ce que** le dispositif comprend en outre
une unité d'établissement de charge (21, 42),
une unité de mesure de mécanomyogramme (25), et
une unité de détermination (33),
dans lequel
l'unité d'établissement de charge (21, 42) établit une charge à imposer à un muscle sur la base de la contraction volontaire maximale calculée,
l'unité de mesure de mécanomyogramme (25) mesure un mécanomyogramme de la personne, et
l'unité de détermination (33) mesure un mécanomyogramme d'un muscle de la personne lorsque la personne fait un exercice par rapport à la charge, analyse les amplitudes et une fréquence moyenne à partir des données chronologiques du mécanomyogramme mesuré, et détermine les proportions des types de muscles dans les parties mesurées de la personne à partir des points d'inflexion des données d'amplitude et des données de fréquence moyenne.

**2.** Dispositif selon la revendication 1, dans lequel les types de muscles déterminés par l'unité de détermination (33) sont une fibre à contraction lente et une fibre à contraction rapide.

**3.** Dispositif selon la revendication 1 ou 2, dans lequel les types de muscles déterminés par l'unité de détermination (33) sont une fibre 50, une fibre FOG et une fibre FG basées sur des différences des propriétés du métabolisme biochimique.

**4.** Dispositif selon l'une quelconque des revendications 1 à 3, dans lequel l'unité d'établissement de charge (21, 42) change la charge par étapes pendant la mesure du mécanomyogramme sur la base de la contraction volontaire maximale calculée.

**5.** Dispositif selon l'une quelconque des revendications 1 à 4, comprenant en outre :

une unité de détection de force musculaire (41),
une unité de commande (33), et
une unité d'affichage (7),
dans lequel
l'unité de détection de force musculaire (41) détecte une force musculaire exercée par la personne pendant la mesure du mécanomyogramme,
l'unité de commande (33) calcule une différence de force musculaire qui est une différence entre la charge établie par l'unité d'établissement de charge (21, 42) et la force musculaire de la personne détectée par l'unité de détection de force musculaire (41), et
l'unité d'affichage (7) affiche la différence de force musculaire.

**6.** Dispositif selon l'une quelconque des revendications 1 à 5, dans lequel l'unité de détermination (33) détermine l'apparition de fatigue musculaire en comparant les données des amplitudes et la fréquence moyenne d'un mécanomyogramme lorsqu'une charge donnée est imposée sur un muscle de la personne avec les données des amplitudes et la fréquence moyenne d'un mécanomyogramme analysé dans le passé.

# FIG. 1

CLASSIFICATIONS OF TYPES OF MUSCLE FIBERS

| SLOW-TWITCH FIBER | FAST-TWITCH FIBER | |
|---|---|---|
| RED MUSCLE FIBER | WHITE MUSCLE FIBER | |
| TYPE I FIBER | TYPE II FIBER | |
| TYPE I FIBER | TYPE II A FIBER | TYPE II B FIBER |
| SO FIBER | FOG FIBER (INTERMEDIATE MUSCLE FIBER) | FG FIBER |

EP 1 413 250 B1

# FIG. 2

15

# FIG. 3

FIG. 4

```
                    ┌──────────────┐
                    │   TURN ON    │
                    │    POWER     │
                    └──────┬───────┘
                           │
                    ┌──────┴───────┐
                    │ INPUT NUMBER │──S1
                    └──────┬───────┘
                           │
         S2                ▼
      NO      ╱ SETTING      ╲
   ┌─────────◄  COMPLETED?   ►
   │          ╲             ╱
 S3│               │ YES
   ▼            S6 ▼
┌─────────┐   YES  ╱          ╲
│INPUT AGE│◄──────◄ SETTING KEY? ►
└────┬────┘        ╲          ╱
     │                  │ NO
     ▼             S7   ▼
┌──────────┐       ╱ MUSCLE TYPE ╲  YES
│  INPUT   │◄─S4  ◄ DETERMINED?   ►──────┐
│ GENDER   │       ╲            ╱        │
└────┬─────┘            │ NO             ▼
     │                  ▼        ┌──────────────┐
┌──────────┐    ┌──────────────┐│DISPLAY AND   │──S8
│INPUT BODY│    │   DETERMINE   ││SELECT WHAT   │
│  HEIGHT  │─S5 │  MUSCLE TYPE  ││TO BE         │
└──────────┘ S10│               ││DETERMINED    │
                └──────────────┘└──────┬───────┘
                                   S9  ▼
                              ╱DETERMINATION OF╲
                         YES ◄  MUSCLE TYPE     ►
                              ╲  SELECTED?     ╱
                                    │ NO  S11
                              ╱DETERMINATION OF╲  NO
                             ◄ MUSCLE FATIGUE   ►────┐
                              ╲  SELECTED?     ╱     │
                                    │ YES            │
                              ┌──────────────┐       │
                              │  DETERMINE   │──S12   │
                              │MUSCLE FATIGUE│       │
                              └──────┬───────┘       │
                              ┌──────▼───────┐       │
                              │   DISPLAY    │──S13   │
                              │   RESULT     │       │
                              └──────┬───────┘       │
                           S14       ▼               │
                              ╱           ╲  YES   ┌────────┐
                             ◄ PRINT KEY?  ►──────►│PRINTING│──S15
                              ╲           ╱        └───┬────┘
                                 │ NO               │
                           S16   ▼                  │
                              ╱           ╲  NO      │
                             ◄ TERMINATION ►────────┐│
                              ╲   KEY?    ╱         ││
                                 │ YES              ││
                           ┌──────▼───────┐
                           │   TURN OFF   │
                           │    POWER     │
                           └──────────────┘
```

# FIG.5

```
        ┌─────────────────┐
        ║ DETERMINATION   ║
        ║   OF MUSCLE     ║
        ║     TYPE        ║
        └─────────────────┘
                 │
  S21   ┌─────────────────┐
        │  INPUT WEIGHT   │
        │  OF CLOTHES     │
        └─────────────────┘
                 │
  S22   ┌─────────────────┐
        │ MEASURE BODY    │
        │    WEIGHT       │
        └─────────────────┘
                 │
  S23   ┌─────────────────┐
        │    DISPLAY      │
        │ INSTRUCTION TO  │
        │  ATTACH CUFF    │
        └─────────────────┘
                 │
  S24      ◇ CUFF          NO
           ◇ ATTACHED? ◇
                 │ YES
  S25   ┌─────────────────┐
        │    MEASURE      │
        │  INPEDANCE OF   │
        │  WHOLE BODY     │
        └─────────────────┘
                 │
  S26   ┌─────────────────┐
        │   CALCULATE     │
        │    INDICES      │
        └─────────────────┘
                 │
  S27   ┌─────────────────┐
        │   CALCULATE     │
        │      MVC        │
        └─────────────────┘
```

```
  S28   ┌─────────────────┐
        │    SET LOAD     │
        └─────────────────┘
                 │
  S29   ┌─────────────────┐
        │    DISPLAY      │
        │ INSTRUCTION TO  │
        │START MEASUREMENT│
        └─────────────────┘
                 │
  S30   ┌─────────────────┐
        │ INCREASE LOAD   │
        │   TO MUSCLE     │
        └─────────────────┘
                 │
        ┌─────────────────┐
        │    MEASURE      │
        │ ELECTROMYOGRAM  │
        │      AND        │
        │ MECHANOMYOGRAM  │
        └─────────────────┘
  S31            │
           ◇  80%           NO
           ◇  MVC?  ◇
  S32            │ YES
        ┌─────────────────┐
        │ LOAD RELEASED AND│
        │ MEASUREMENT OF  │
        │ MECHANOMYOGRAM  │
        │   COMPLETED     │
        └─────────────────┘
  S33
```

```
  S34   ┌─────────────────┐
        │    ANALYZE      │
        │   FREQUENCY     │
        └─────────────────┘
                 │
  S35   ┌─────────────────┐
        │   CALCULATE     │
        │AVERAGE FREQUENCY│
        │AND RMS AMPLITUDE│
        └─────────────────┘
                 │
  S36   ┌─────────────────┐
        │   CALCULATE     │
        │INFLECTION POINTS│
        └─────────────────┘
                 │
  S37   ┌─────────────────┐
        │   DETERMINE     │
        │  MUSCLE TYPE    │
        └─────────────────┘
                 │
  S38   ┌─────────────────┐
        │  STORE RESULTS  │
        └─────────────────┘
                 │
           ╭─────────╮
           │  EXIT   │
           ╰─────────╯
```

# FIG. 6

DETERMINATION
OF MUSCLE
FATIGUE

S41 — DISPLAY
INSTRUCTION TO
ATTACH CUFF

S42 — CUFF
ATTACHED? —— NO

YES

S43 — SET 20% LOAD

S44 — DISPLAY
INSTRUCTION TO
START MEASUREMENT

S45 — MEASURE
ELECTROMYOGRAM
AND
MECHANOMYOGRAM

S46 — 10 SECONDS
ELAPSED ? —— NO

YES

S47 — LOAD RELEASED
AND MEASUREMENT
COMPLETED

S48 — ANALYZE FREQUENCY

S49 — CALCULATE AVERAGE
FREQUENCY AND
RMS AMPLITUDE

S50 — COMPARE WITH
REFERENCE DATA

S51 — DETERMINE MUSCLE
FATIGUE

EXIT

# FIG. 7

DISPLAY MUSCLE        PRINT

SEX : MALE
AGE : 25
HEIGHT : 175 cm
BODY WEIGHT : 56 kg
BODY FAT PERCENTAGE : 22 %
FAT FREE MASS : 34 kg
BMI: 18.3

# FIG. 8

BODY FAT PERCENTAGE        PRINT

MAXIMUM MUSCULAR STRENGTH : 43 kg

TYPE OF MUSCLE
        SO : 45 %    SLOW-TWITCH FIBER : 45 %
        FOG : 25 % ⎫
        FG : 30 % ⎭ FAST-TWITCH FIBER : 55 %

MUSCLE FATIGUE : 30 %

## FIG. 9

## FIG. 10

# FIG. 11

# FIG. 12

FREQUENCY OF
EMISSION OF MU

⬚ LOW REGION
⬚ MEDIUM REGION
⊞ HIGH REGION

INFLECTION POINTS AND ACTION
PATTERNS OF MUSCLE FIBERS

Ra: SLOW-TWITCH FIBERS ACT

Rb: SWITCHING FROM SLOW-TWITCH FIBERS
TO FAST-TWITCH FIBERS

Rc: FAST-TWITCH FIBERS PREDOMINANTLY
MOBILIZED

Rd: FAST-TWITCH FIBERS (TYPE II b)

Re: ALL FIBERS MOBILIZED

EP 1 413 250 B1

# FIG. 13

SWITCH

PRINT

**UPPER BODY**

MAXIMUM MUSCULAR
STRENGTH                    43kg

TYPE OF MUSCLE
SO : 45%        SLOW-TWITCH FIBER : 45%
FOG : 25%
FG : 30%        FAST-TWITCH FIBER : 55%

MUSCLE FATIGUE    30%

**LOWER BODY**

MAXIMUM MUSCULAR
STRENGTH                    43kg

TYPE OF MUSCLE
SO : 45%        SLOW-TWITCH FIBER : 45%
FOG : 25%
FG : 30%        FAST-TWITCH FIBER : 55%

MUSCLE FATIGUE    30%

EP 1 413 250 B1

# FIG. 14

| BODY FAT PERCENTAGE | DISPLAY MUSCULAR STRENGTH | PROPORTIONS OF TYPES OF MUSCLES | PRINT |

| | | RIGHT HAND | LEFT HAND | RIGHT FOOT | LEFT FOOT |
|---|---|---|---|---|---|
| MEASURED MUSCULAR (kg) | | 20 | 25 | 20 | 22 |
| STRENGTH TYPE OF MUSCLE | SO (%) | 45 | 40 | 44 | 42 |
| | FOG (%) | 35 | 37 | 31 | 37 |
| | FG (%) | 20 | 23 | 25 | 21 |

# FIG. 15

| BODY FAT PERCENTAGE | DISPLAY MUSCULAR STRENGTH | PROPORTIONS OF TYPES OF MUSCLES | PRINT |

■ MAXIMUM MUSCULAR STRENGTH

LEFT FOOT

RIGHT FOOT

LEFT HAND

RIGHT HAND

0    20    40    60    80    100    120

## FIG. 16

| | BODY FAT PERCENTAGE | DISPLAY MUSCULAR STRENGTH | PROPORTIONS OF TYPES OF MUSCLES | PRINT |

Legend: □ SO  ⊠ FOG  ■ FG

LEFT FOOT

RIGHT FOOT

LEFT HAND

RIGHT HAND

0%  20%  40%  60%  80%  100%